(19) Europäisches Patentamt
European Patent Office
Office européen des brevets

(11) Publication number : **0 437 347 B1**

# (12) EUROPEAN PATENT SPECIFICATION

(45) Date of publication of patent specification : **13.04.94 Bulletin 94/15**

(51) Int. Cl.$^5$ : **A61K 7/075,** A61K 7/50

(21) Application number : **91300143.4**

(22) Date of filing : **09.01.91**

## (54) Topical composition.

(30) Priority : **12.01.90 GB 9000704**

(43) Date of publication of application : **17.07.91 Bulletin 91/29**

(45) Publication of the grant of the patent : **13.04.94 Bulletin 94/15**

(84) Designated Contracting States : **AT BE CH DE DK ES FR GB GR IT LI NL SE**

(56) References cited :
EP-A- 0 018 717
EP-A- 0 024 799
EP-A- 0 077 920
EP-A- 0 371 801
EP-A- 0 371 802
EP-A- 0 371 803
EP-A- 0 371 804
GB-A- 2 027 047
US-A- 4 758 376

(73) Proprietor : **UNILEVER PLC**
**Unilever House Blackfriars P.O. Box 68**
**London EC4P 4BQ (GB)**
(84) **GB**
Proprietor : **UNILEVER N.V.**
**Weena 455**
**NL-3013 AL Rotterdam (NL)**
(84) **BE CH DE DK ES FR GR IT LI NL SE AT**

(72) Inventor : **Birtwistle, David Howard**
**Quarry Road East, Bebington**
**Wirral, Merseyside L63 3JW (GB)**
Inventor : **Carter, Peter**
**Quarry Road East, Bebington**
**Wirral, Merseyside L63 3JW (GB)**
Inventor : **Rosser, David Arthur**
**Quarry Road East, Bebington**
**Wirral, Merseyside L63 3JW (GB)**

(74) Representative : **Barker, Rosemary Anne et al**
**c/o Mewburn Ellis 2 Cursitor Street**
**London EC4A 1BQ (GB)**

**EP 0 437 347 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to compositions suitable for topical application to the skin, (including the mucosae), and to the hair. In particular, the invention is concerned with highly improved detergent compositions suitable for cleansing the whole body surface, including the mouth.

The damaging effect of conventional detergents used to wash the body surface, particularly where young, tender or damaged skin is involved, has been the subject of intense study for many years in a search for milder-to-the-skin products, which not only cleanse the skin efficiently, but also leave the skin with a pleasant smooth silky feel after the skin surface has been dried off.

The use of certain mono- and di-alkyl phosphate salts for this purpose has been advocated in view of their mild characteristics, but some of this group of salts are used as antifoam agents because of their lather suppressant properties, and would therefore require careful formulation if lather control is not required.

To this end, US patent No. 4,139,485 (Kao Soap Co. Ltd.) describes a detergent composition having low irritation properties on human skin, wherein the surfactant component is dialkyl or dialkenyl phosphate salt (DAP) and/or monoalkyl or monoalkenyl phosphate salt (MAP), each alkyl or alkenyl group having from 10 to 16 carbon atoms), the weight ratio of 'DAP' to 'MAP' being from 20:80 to 0:100. This system is stated to possess good detergency.

Also, in US patent No. 4,526,710 Kao Corporation report a study to improve properties of detergent composition which make use of anionic phosphate surface active agents, which Kao maintain are highly innoxious and particularly mild to the skin. As a result, Kao have found that when phosphate ester salts having a specific ion pair, notably mono- or di-alkyl (C8-18) phosphates, are used in combination with alkanol amine salts of higher fatty acids and alkyl amine oxides, the detergency and foaming characteristics are remarkably improved.

Also, US patent No. 4,758,376 (Kao) discloses an alternative composition, comprising an alkanolamine salt of a mono or dialkyl (C8-18) phosphate or mixtures thereof, to that described in US patent No. 4,526,710 in which the problem of poor foaming due to the dialkyl phosphate salt is dealt with by incorporating with the phosphate a compound chosen from an amidoamine amphoteric surfactant or hydroxysulphobetaine, or an aliphatic lactylate or glycolate.

It is clear from a study of the three Kao references, that all the evidence on which their disclosures are based is restricted to alkyl phosphates or dialkyl phosphates in which each alkyl group has at least 10 carbon atoms. Furthermore, the two alkyl groups of the dialkyl phosphate each comprise the same number of carbon atoms and are each otherwise identical with each other.

As further prior art there may be mentioned EP-A-0371801, EP-A-0371802, EP-A-0371803 and EP-A-0371804 (all of Unilever), all of which references form part of the state of the art under Article 54(3) EPC. Each of these four references discloses aqueous compositions for topical application to skin or hair, which comprise, inter alia, certain mild, good foaming phosphate surfactants which include two groups each chosen from branched or unbranched alkyl or alkenyl groups having an average of from 7 to 9 carbon atoms.

Applicants in their search for a mild surfactant for use in cleansing human skin or hair, with the added attribute that a full, soft lather is produced without necessarily incorporating a secondary surfactant such as those proposed by Kao, have unexpectedly discovered that certain dialkyl phosphate salts, in which the alkyl groups are different from each other, meet their requirements in this respect. Also, the composition containing these unusual "asymmetrical" alkyl phosphate salts is accordingly capable of producing a superior lather volume and an outstanding lather creaminess. The composition is, furthermore, so mild to the skin that it can safely be used for cleansing the mucosae, such as the mouth and the vagina, and other more delicate skin areas. It can also be used in shampoos for frequent, e.g. daily, hair washing, without risk of scalp irritation or damage attributable to harsher products. In addition to these excellent attributes, the ease of rinsing from hair or skin and superior silky-smooth after-use skin feel properties of the compositions, including freedom from skin roughness and erythema, have great consumer appeal.

### DEFINITION OF THE INVENTION

Accordingly, the invention provides a composition suitable for topical application to the skin or hair, which comprises:

(a) from 1 to 99.9% by weight of phosphate surfactant having the structure (1):

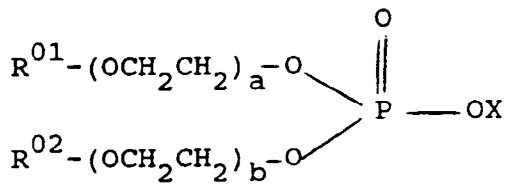

(1)

where $R^{01}$ and $R^{02}$ are different from each other and are each chosen from branched or unbranched alkyl and alkenyl groups having an average of from 1 to 16 carbon atoms, the total number of carbon atoms present in the alkyl and alkenyl groups being from 10 to 20;

X is chosen from H, alkali metal ammonium and substituted ammonium counterions; and

a & b are each chosen from 0 or a value of from 1 to 4;

but not including those phosphate surfactants which are n-hexyl-n-nonyl phosphate and n-hexyl-n-octyl phosphate; and

(b) from 0.01 to 99% by weight of a cosmetically acceptable vehicle for the surfactant.

## DISCLOSURE OF THE INVENTION

### The Phosphate Surfactant

The composition according to the invention comprises "asymmetrical" phosphate surfactant having the structure (1):

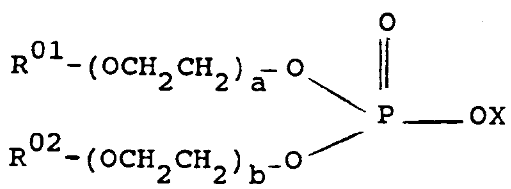

(1)

where $R^{01}$ and $R^{02}$ are different from each other and are each chosen from branched or unbranched alkyl and alkenyl groups having an average of from 1 to 16 carbon atoms, the total number of carbon atoms present in the alkyl or alkenyl groups being from 10 to 20;

X is chosen from H, alkali metal ammonium and substituted ammonium counterions; and

a & b are each chosen from 0 or a value of from 1 to 4.

Excluded from the above class of phosphate surfactants are those which are n-hexyl-n-nonyl phosphate and n-hexyl-n-octyl phosphate

Preferably, the number of carbon atoms present in the alkyl or alkenyl group $R^{01}$ is from 12 to 16, and the number of carbon atoms present in the alkyl or alkenyl group $R^{02}$ is from 1 to 4.

Examples of the "asymmetrical" phosphate moiety of the phosphate surfactant include:

n-butyl-lauryl phosphate
n-hexyl-decyl phosphate
ethyl-myristyl phosphate
methyl-lauryl phosphate
n-octenyl-n-nonenyl phosphate
ethyl-lauryl phosphate, and
ethyl-dodecenyl phosphate.

The preferred counterion is chosen from sodium, potassium and triethanolammonium.

The phosphate surfactant will normally comprise mixtures of dialkyl and/or dialkenyl and/or alkylalkenyl, surfactants, including also some monoalkyl or monoalkenyl phosphate surfactants, as preparation of pure phosphate surfactant without a least a trace of related phosphate surfactant is difficult.

The amount of "asymmetrical" phosphate surfactant which is present in the composition according to the invention is from 1 to 99.9%, preferably from 2 to 50% by weight of the composition.

Compositions containing less than 1% by weight of "asymmetrical" phosphate surfactant show poor lather characteristics and lack the desired feel properties associated with compositions containing higher levels of this ingredient.

The Cosmetically Acceptable Vehicle

The selection of a suitable vehicle will depend on the required product form of the composition. Typically, the vehicle will be chosen from diluents, dispersants or carriers for the phosphate surfactant, so as to ensure an even distribution of it when applied to the skin.

Compositions according to this invention can include water as a vehicle, usually with at least one other cosmetically-acceptable vehicle.

Vehicles other than water that can be used in compositions according to the invention can include liquids or solids as emollients, solvents, humectants, thickeners and powders. Examples of each of these types of vehicles, which can be used singly or as mixtures of one or more vehicles, are as follows:

Emollients, such as stearyl alcohol, glyceryl monolaurate, glyceryl monoricinoleate, glyceryl monostearate, propane-1,2-diol, butane-1,3-diol, docosan-1,2-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polythylene glycol, triethylene glycol, lanolin, cocoa butter, corn oil, cotton seed oil, tallow, lard, olive oil, palm kernel oil, rapeseed oil, safflower seed oil, soybean oil, sunflower seed oil, olive oil, sesame seed oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

Propellants, such as trichlorofluoromethane, dichlorodifluoromethane, dichlorotetrafluoroethane, monochlorodifluoromethane, trichlorotrifluoroethane, propane, butane, isobutane, dimethyl ether, carbon dioxide, nitrous oxide;

Solvents, such as ethyl alcohol, methylene chloride, isopropanol, acetone, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 0.01 to 99.9%, preferably from 50 to 98% by weight of the composition, and can, in the absence of other cosmetic adjuncts, form the balance of the composition.

Water

The composition according to the invention can also comprise water, which can also act as a vehicle for the phosphate surfactant and to enable it to be provided at a concentration suitable for convenient topical application to human skin.

The amount of water present in the composition of the invention is accordingly up to 99%, preferably from 5 to 99% by weight of the composition.

Co-surfactant

The composition according to the invention can also optionally comprise a co-surfactant, further to modify the surfactant properties attributable to the phosphate surfactant.

Examples of co-surfactants include anionic surfactants other than the phosphate surfactants defined herein, as well as nonionic, amphoteric and zwitterionic surfactants.

Anionic Co-surfactants

Particularly preferred co-surfactants, when employed, are anionic surfactants, examples of which are set out hereinafter.

i. Short Chain "Symmetrical" Phosphate Surfactant

The composition according to the invention can optionally comprise as a co-surfactant, a short chain phosphate surfactant having the structure (2a):

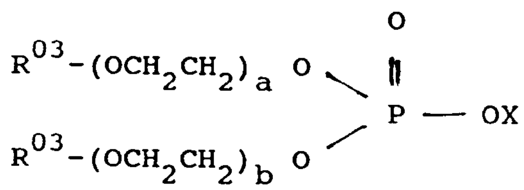

(2a)

where the $R^{03}$ groups are the same and are each chosen from branched or unbranched alkyl and alkenyl groups having an average of from 7 to 9 carbon atoms;

X is chosen from H, alkali metal ammonium and substituted ammonium counterions; and

a & b are each chosen from 0 or a value of from 1 to 4.

Examples of the phosphate moiety of the short chain symmetrical phosphate surfactant include:

di-n-heptyl phosphate
di-n-octyl phosphate
di-n-nonyl phosphate
di-(diethyleneglycol-mono-n-nonyl ether) phosphate
di-(ethyleneglycol-mono-n-octyl ether) phosphate
di-n-heptenyl phosphate
di-n-octenyl phosphate
di-n-nonenyl phosphate
di-(triethyleneglycol-mono-n-octenyl ether) phosphate
di-(ethyleneglycol-mono-n-heptyl ether) phosphate
di-(7-methyloctyl) phosphate
di-(5-methylhexyl) phosphate
di-(6,6-dimethylheptyl) phosphate
n-hexyl-n-nonyl phosphate, and
n-hexyl-n-octyl phosphate.

The preferred short chain symmetrical phosphate moiety is di-n-octyl phosphate, and the preferred counterion is chosen from sodium, potassium and triethanolammonium.

The amount of short chain symmetrical phosphate surfactant which can optionally be present in the composition according to the invention is up to 99%, preferably from 2 to 50% by weight of the composition.

ii. Monoalkyl or Monoalkenyl Phosphate Surfactants

The composition according to the invention can optionally also comprise, as a co-surfactant, a monoalkyl or monoalkenyl phosphate surfactant having the structure (2b):

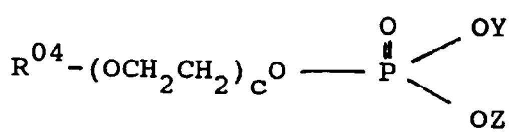

(2b)

where $R^{04}$ is chosen from branched or unbranched alkyl and alkenyl groups having from 6 to 18 carbon atoms;

Y and Z are each chosen from H, alkali metal, ammonium and substituted ammonium counterions; and

c is chosen from 0 or a value of from 1 to 4.

Examples of the phosphate moiety of the monoalkyl and monoalkenyl phosphate surfactant include:

mono-n-hexyl phosphate
mono-n-heptyl phosphate
mono-n-octyl phosphate
mono-n-nonyl phosphate
mono-n-decyl phosphate
mono-n-dodecyl phosphate (mono-lauryl phosphate)

mono-n-tetradecyl phosphate (monomyristyl phosphate)
mono-n-hexadecyl phosphate
mono-n-octadecyl phosphate
mono-(diethyleneglycol-mono-n-nonyl ether) phosphate
mono-(ethyleneglycol-mono-n-octyl ether) phosphate
mono-n-hexenyl phosphate
mono-n-heptenyl phosphate
mono-n-octenyl phosphate
mono-n-nonenyl phosphate
mono-n-dodecenyl phosphate
mono-(triethyleneglycol-mono-n-octenyl ether) phosphate
mono-(ethyleneglycol-mono-n-heptenyl ether) phosphate
mono-7-methyloctyl phosphate
mono-5-methylhexyl phosphate
mono-6,6-dimethylheptyl phosphate
mono-(ethyleneglycol-mono-n-octadecyl ether) phosphate
mono-(diethyleneglycol-mono-n-octadecenyl ether) phosphate
mono-(polyethyleneglycol[5EO]-monooleyl ether) phosphate, and
mono-(polyethyleneglycol[3EO]-monolauryl ether) phosphate.

Preferably, the $R^{04}$ group is lauryl ($C_{12}$), the monoalkyl phosphate surfactant being a monolauryl phosphate triethanolamine salt.

The amount of the monoalkyl or monoalkenyl phosphate surfactant which can be present in the composition according to the invention is up to 99%, preferably from 2 to 50% by weight of the composition.

iii. <u>Long Chain "Symmetrical" Dialkyl or Dialkenyl Phosphate Surfactant</u>

The composition according to the invention can also optionally comprise a long chain dialkyl or dialkenyl phosphate surfactant having the structure (2c):

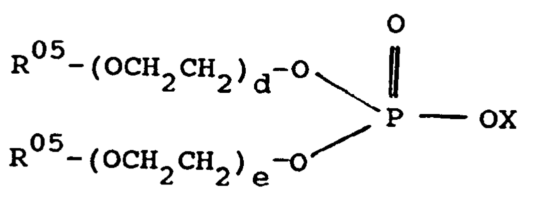

(2c)

where the $R^{05}$ groups are the same and are each chosen from branched or unbranched alkyl and alkenyl groups having an average of from 10 to 18 carbon atoms;

X is chosen from H, alkali metal ammonium and substituted ammonium counterions; and

d and e are each chosen from 0 or a value of from 1 to 10.

Examples of the phosphate moiety of the long chain dialkyl and dialkenyl phosphate surfactant include:

di-n-decyl phosphate
di-n-dodecyl phosphate (dilauryl phosphate)
di-n-tetradecyl phosphate (dimyristyl phosphate)
di-n-hexadecyl phosphate
di-n-octadecyl phosphate
di-n-dodecenyl phosphate
di-(ethyleneglycol-mono-n-octadecenyl ether) phosphate
di-(diethyleneglycol-mono-n-octadecenyl ether) phosphate
di-(polyethyleneglycol[5EO]-monooleyl ether) phosphate, and
di-(polyethyleneglycol[3EO]-monolauryl ether) phosphate.

The preferred long chain dialkyl phosphate surfactant, when present, is triethanolammonium dilauryl phosphate.

The amount of the long chain dialkyl or dialkenyl phosphate surfactant, when present, is up to 20%, preferably from 1 to 10% by weight, of the composition.

The weight ratio of the monoalkyl or monoalkenyl phosphate surfactant to the long chain dialkyl phosphate or dialkenyl surfactant, when both are present, is preferably from 100:0 to 50:50, most preferably 95:5 to 75:25.

iv. Fatty acid soap co-surfactant

The composition according to the invention can optionally comprise, as a co-surfactant one or more soaps which are water-soluble or water-dispersable alkali metal salts of an organic acid, especially a sodium or a potassium salt, or the corresponding ammonium or substituted ammonium salt. Examples of suitable organic acids are natural or synthetic alkanoic acids having from 10 to 22 carbon atoms, especially the fatty acids of triglyceride oils such as tallow and coconut oil.

For solid products, such as powders, bars or tablets, the preferred soap is a soap of tallow fatty acids, that is fatty acids derived from tallow class fats, for example beef tallow, mutton tallow, lard, palm oil and some vegetable butters. Minor amounts of up to about 30%, preferably 10 to 20%, by weight of sodium soaps of nut oil fatty acids derived from nut oils, for example coconut oil and palm kernel oil, may be admixed with the sodium tallow soaps, to improve their lathering and solubility characteristics if desired. Whereas tallow fatty acids are predominantely $C_{14}$ and $C_{18}$ fatty acids, the nut oil fatty acids are of shorter chain length and are predominantly $C_{10}$-$C_{14}$ fatty acids.

For liquid or gel products, the preferred soaps are predominantely $C_{10-14}$ fatty acids derived from nut oils,or alternatively, from synthetic alkanoic acids.

The soaps can be provided as a preformed ingredient for the composition, or they can be formed in situ during the manufacture of the composition by reaction of suitable fatty acids and an alkali.

The amount of fatty acid soap which can be present in the composition according to the invention is up to 90%, preferably from 2 to 80% by weight of the composition.

v. Non-soap anionic co-surfactants

The composition according to the invention can also optionally comprise one or more non-soap anionic co-surfactants, examples of which include:

The alkali metal salts of organic sulphuric reaction products having an alkyl or acyl radical containing from 8-22 carbon atoms and a sulphonic acid or sulphuric acid ester group. Specific examples of these synthetic anionic surfactants are the sodium, ammonium, potassium or triethanolammonium alkyl sulphates, especially those obtained by sulphating the higher alcohols ($C_8$-$C_{18}$), sodium coconut oil fatty acid monoglyceride sulphates and sulphonates; sodium or potassium salts of sulphuric esters of the reaction product of one mole of a higher fatty alcohol (e.g. tallow or coconut oil alcohols) and 1-12 moles of ethyleneoxide; sodium or potassium salts of alkyl phenol ethylene oxide ether sulphate with 1-10 units of ethylene oxide per molecule and in which the alkyl group contains from 8 to 12 carbon atoms, sodium alkyl glyceryl ether sulphonates, the reaction product of fatty acids having from 10 to 22 carbon atoms esterified with isethionic acid and neutralised with sodium hydroxide; water soluble salts of condensation products of fatty acids with N-methyl taurine.

Especially preferred non-soap anionic co-surfactants include:

alkylaryl sulphonates, such as sodium alkyl benzene sulphonate (e.g. TEEPOL CM44, available from Shell).

alkyl sulphates, such as sodium lauryl sulphate (e.g. EMPICOL CX, available from Albright & Wilson), and triethanolammonium lauryl sulphate (e.g. EMPICOL TL40/T, available from Albright & Wilson).

alkylether sulphates, such as sodium lauryl ether sulphate (e.g. EMPICOL ESB70, available from Albright & Wilson).

alkyl sulphonates, such as sodium alkane (C13-18) sulphonate (e.g. HOSTAPUR SAS 30, available from Hoechst).

olefin sulphonates, such as sodium olefin sulphonate (C15-18) (e.g. HOSTAPUR OS,available from Hoechst).

Sarcosinates, having the structure (3):

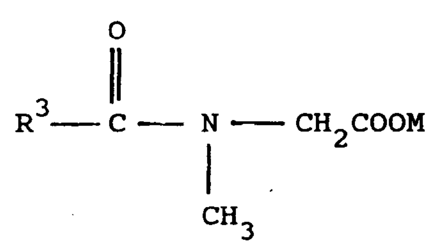

(3)

where $R^3$ is chosen from $C_{6-14}$ alkyl, and

M is a counterion chosen from alkali metals, ammonium, substituted ammonium, such as alkanolammonium.

An example of sarcosinates having the structure (3),

sodium lauryl sarcosinate (e.g. HAMPOSYL L-95, available from Grace).

Taurides, having the structure (4):

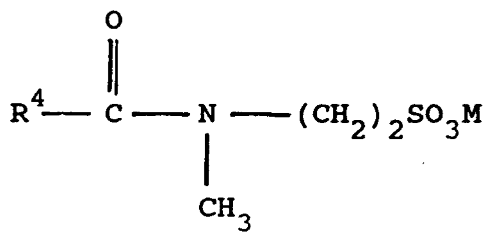

(4)

where $R^4$ is chosen from $C_{8-18}$ alkyl

An example of taurides having the structure (4) is:

coconut methyl taurine (e.g. FENOPON TC 42, available from GAF).

Isethionates, having the structure (5):

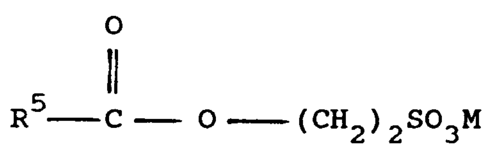

(5)

where $R^5$ is chosen from $C_{8-18}$ alkyl.

An example of isethionates having the structure (5) is:

sodium acyl isethionate (e.g. JORDAPON C1, available from Jordan).

Monoalkyl sulphosuccinates, having the structure (6):

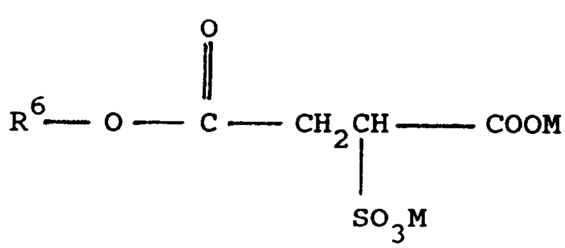

(6)

where $R^6$ is chosen from $C_{10-20}$ alkyl.

Examples of monoalkyl sulphosuccinates having this structure (6) include:
sodium lauryl sulphosuccinate (e.g. EMPICOL SLL, available from Albright & Wilson)
magnesium alkyl sulphosuccinate (e.g. ELFANOL 616 Mg, available from AKZO),
sodium lauryl ethoxysulphosuccinate (e.g. EMPICOL SDD, available from Albright & Wilson)
coconut monoethanolamide ethoxysulphosuccinate, (e.g. EMPICOL SGG)
disodium lauryl polyglycolether sulphosuccinate (e.g. SURTAGENE S30, available from CHEM-Y)
polyethyleneglycol sulphosuccinate (e.g. REWOPOL SBFA 30, available from REWO).

Dialkyl sulphosuccinates, having the structure (7):

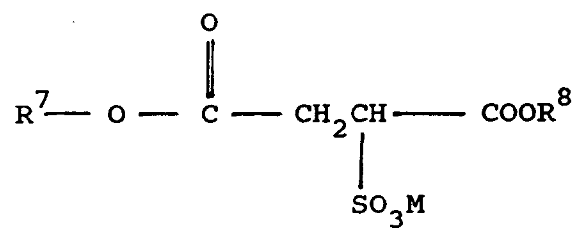

(7)

where $R^7$ and $R^8$ are the same or different, and are are chosen from $C_{6-14}$ alkyl.

An example of dialkyl sulphosuccinate having the structure (7) is:
sodium dioctyl sulphosuccinate (e.g. EMCOL 4500 available from Witco).

Acyl lactylates, having the structure (8):

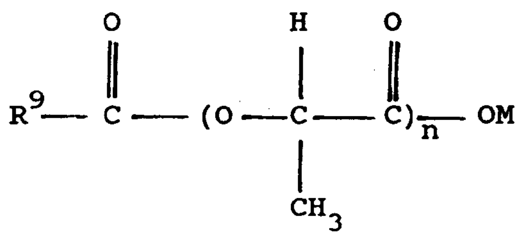

(8)

where $R^9$ is chosen from $C_{6-16}$ alkyl, and
n is 1 or 2

An example of acyl lactylates having the structure (8) is:
decanoyl lactylate (e.g. PATIONIC 122A, available from Patterson, C.J.).

Acylatedα-amino acids, such as sodium lauroyl glutamate (e.g. ACYLGLUTAMATE LS-11, available from Ajinomoto Co. Inc.).

Ethyl carboxylates, such as alkyl $C_{12-14}O(EO)_4OCH_2CO_2Na$ (e.g. AKYPO RLM 38, available from AKZO).

## Nonionic co-surfactants

A composition according to the invention can also comprise nonionic co-surfactants which are compounds produced by the condensation of alkylene oxide groups (hydrophilic in nature) with an organic hydrophobic compound, which may be aliphatic or alkyl aromatic in nature. Examples of nonionic co-surfactants include:

i. The polyethylene oxide condensates of alkyl phenols having from 6 to 12 carbon atoms, either straight or branched chain, with ethylene oxide, which is present in amounts of from 10 to 60 moles of ethylene oxide per mole of alkylphenol.

ii. The condensation product of aliphatic alcohols having from 8 to 18 carbon atoms, straight or branched chain, with ethyleneoxide, for example, a coconut alcohol ethyleneoxide condensate having from 10 to 13 moles of ethylene oxide per mole of coconut alcohol, the coconut alcohol fraction having from 10 to 14 carbon atoms.

iii. Long chain tertiary amineoxides having the structure (9):

$$R^{10}R^{11}R^{12}N \rightarrow O \qquad (9)$$

where $R^{10}$ contains an alkyl, alkenyl or monohydroxyalkyl radical of from 8 to 18 carbon atoms, from 0 to 10 ethylene oxide moieties and from 0 to 1 glyceryl moiety, and $R^{11}$ and $R^{12}$ contain from 1 to 3 carbon atoms and up to 1 hydroxy group, for example, methyl, ethyl, propyl, hydroxyethyl, or hydroxypropyl groups.

Especially preferred examples of nonionic cosurfactants include:
alkylethoxylates, such as the DOBANOL series, available from Shell;
esterethoxylates, such as the TAGAT series, available from Goldschmidt;
alkylalkanolamides, such as coconut monoethanolamide (e.g. EMPILAN CME, available from Albright & Wilson), and coconut diethanolomide, (e.g. EMPILAN CDE, available from Albright & Wilson).
sugar esters, such as sucrose laurate and methyl glucose laurate (available from Grillo-Werke A.G.)
esters of glycols, such as ethylene glycol mono stearate.

esters of glycerol, such as glyceryl mono stearate.
ethoxylated sorbitan esters, such as the TWEEN series (available from ICI).
amine oxides, such as alkyldimethyl amine oxide (e.g. EMPIGEN OB, available from Albright & Wilson) and alkylethoxydimethyl amine oxide (e.g. EMPIGEN OY, available from Albright & Wilson).

Zwitterionic and Amphoteric co-surfactants

The composition according to the invention can also contain zwitterionic co-surfactants, which are derivatives of aliphatic quaternary ammonium, phosphonium and sulphonium compounds in which the aliphatic radicals can be straight or branched chain, and where one aliphatic substituent contains from 8 to 18 carbon atoms, and one contains an anionic water-solubilising group, such as carboxyl, sulphonate, sulphate, phosphate or phosphonate.
Examples of zwitterionic co-surfactant include:
4-[N,N-di(2-hydroxyethyl)-N-octadecylammonio]butane-1-carboxylate, and
5-N,N-di(3-hydroxypropyl)-N-hexadecylammonio]-2-hydroxypentane-1-sulphate.
Particularly preferred zwitterionic co-surfactants are betaines, preferred examples of which are:
Alkyl betaines, having the structure (10):

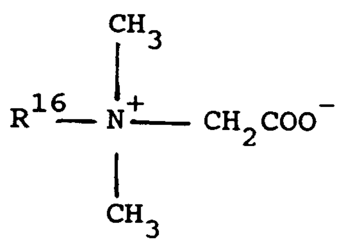

(10)

where $R^{16}$ is $C_{10-16}$ alkyl.
An example of alkyl betaines having the structure (10) is:
lauryldimethyl betaine (e.g. EMPIGEN BB, available from Albright & Wilson).
Alkylamidopropyl betaines, having the structure (11):

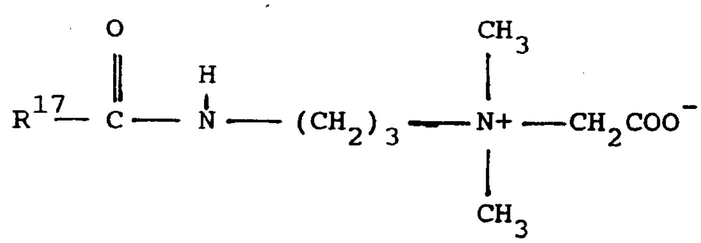

(11)

where $R^{17}$ is $C_{10-16}$ alkyl.
An example of alkylamidopropyl betaines having the structure (11) is:
cocamidopropyl betaine (e.g. TEGOBETAIN L7, available from Goldschmidt).
Alkylamphoglycinates, and having the structure (12):

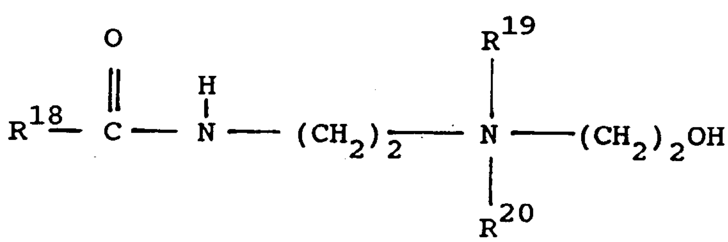

(12)

where $R^{18}$ is $C_{10-16}$ alkyl

$R^{19}$ and $R^{20}$ are the same or different and are chosen from H, $CH_2COO^-$ and $(CH_2)_2COO^-$

An example of alkylamphoglycinerates having the structure (12) is:
cocoamphoglycinate (available from GAF), and alkoamphodipropionate.

Sultaines, having the structure (13):

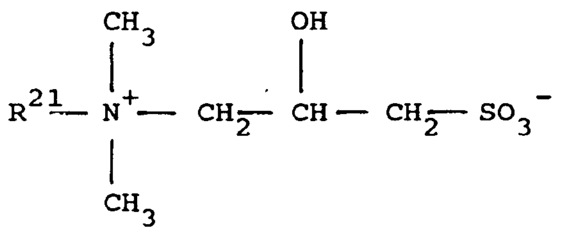

(13)

where $R^{21}$ is chosen from $C_{12-16}$ alkyl or alkylamido.

An example of sultaines having the structure (13) is:
cocoamidopropylhydroxysultaine (e.g. CYCLOTERIC BET-CS, available from Alcolac).

Particularly preferred examples of amphoteric co-surfactants include:
Alkoamphoacetates, such as cocoamphoacetate (e.g. MIRANOL CM), and
Alkoamphopropionates, such as cocoamphopropionate (e.g. MIRANOL CM-SF)
both available from Miranol Inc.

The amount of co-surfactant when present in the compositions according to the invention is usually up to 50%, preferably from 1 to 40% by weight.

Optional thickening agent

The composition according to the invention can also comprise a polymer thickener in an amount sufficient to adjust the viscosity of the composition, so as to facilitate dispensing it conveniently onto the body surface.

Examples of polymer thickeners include:

anionic cellulose materials, such as sodium carboxy methyl cellulose;
anionic polymers such as carboxy vinyl polymers, for examples Carbomer 940 and 941;
nonionic cellulose materials, such a methyl cellulose and hydroxy propyl methyl cellulose;
cationic cellulose materials, such as Polymer JR 400;
cationic gum materials, such as Jaguar C13 S;
other gum materials such as gum acacia, gum tragacanth, locust bean gum, guar gum and carrageenan;
proteins, such as albumin and protein hydrolysates; and
clay materials, such as bentonite, hectorite, magnesium aluminium silicate, sodium magnesium silicate and a synthetic complex clay having the generic formula:
$[Si_8Mg_{5.1}Li_{0.6}H_{4.6}0_{24}]^{0.6-}$ $Na+_{0.6}$, an example of which is Laponite, available from Laporte Industries.

The amount of thickening agent which can optionally be employed in the composition according to the invention is normally from 0.05 to 5%, preferably 0.1 to 1% by weight of the composition.

Preservative

The composition according to the invention can also optionally comprise a preservative to prevent microbial spoilage, especially biodegradation of the alkyl phosphate salt. It is accordingly apparent that the composition containing the alkyl phosphate salt may be prone to attack by bacteria, moulds and fungi and other microbial influences. There is therefore a risk that the shelf-life of the composition might be unacceptably short due to the biodegradation or spoilage, unless there is included in the composition a bactericide, fungicide or other microbicide in an amount sufficient to inhibit or prevent the said biodegradation or spoilage, or unless other steps are taken to preserve the composition.

Examples of preservatives include:

(i) Chemical preservatives, such as ethanol, benzoic acid, sodium benzoate, sorbic acid, potassium sorbate, sodium propionate and the methyl, ethyl, propyl and butyl esters of p-hydroxybenzoic acid 2-bromo-2-nitropropane-1,3-diol, phenoxyethanol, dibromodicyanobutane, formalin and Triclosan. The amount of

chemical preservative optionally to be incorporated in the composition according to the invention will generally be from 0.05 to 5%, preferably from 0.1 to 2% by weight, the amount chosen being sufficient to arrest microbial proliferation.

(ii) Water activity depressants, such as glycerol, propylene glycol, sorbitol, sugars and salts, for examples alkali metal halides, sulphates and carboxylates. When employing a water activity depressant, sufficient should be incorporated in the composition according to the invention to reduce the water activity ($\alpha_w$) from 1 to <0.9, preferably to <0.85 and most preferably < 0.8, the lowest of these values being that at which yeasts, moulds and fungi will not proliferate.

Further optional ingredients

The composition according to the invention can also contain other optional adjuncts, that is ingredients other than the main ingredients already defined which are conventionally employed in compositions for topical application to human skin. Some of these adjuncts, can also function as cosmetically acceptable vehicles and, when present, will normally form the balance of the composition.

Examples of further optional adjuncts include: natural moisturising factors, such as lactic acid, pyrrolidone carboxylic acid or urea; foam controllers; UV absorbers; colourants; and pearlescent agents.

## PRODUCT FORM OF THE COMPOSITION

The composition according to the invention can take the form of a liquid or gel, intended to be dispensed from a capped container such as a bottle, roll-on applicator or tube, or a pump-operated or propellant-driven aerosol dispenser, as a skin cleanser, shower product, bath additive or shampoo. The composition can also take for form of a powder or a solid such as a stick, preferably housed in a suitable capped holder with a wind-up or push-up action similar to a lip stick, or a bar or tablet, with or without fatty acid soaps, intended to be used for washing instead of a conventional soap bar.

The invention also provides a closed container containing a detergent composition as herein defined.

### Process for Preparing the Composition

The invention also provides a process for preparing the composition of the type defined herein, which process comprises the steps of:

(i) preparing a mixture comprising one or more "asymmetrical" phosphate surfactants, as defined herein, and one or more cosmetically acceptable vehicles as defined herein, and

(ii) subsequently packaging the mixture into containers.

### Use of the composition

The composition according to the invention is intended primarily as a personal washing product for cleansing the face and other sensitive parts of the body surface, including the mucosae. It can also be used for washing the hair as well as the skin.

In use, a small quantity, for example from 1 to 5 ml, of the composition is either rubbed between the hands, together with water to form a foam, which is then used for washing, or applied via a flannel or sponge to the area to be cleansed, or the foam is generated directly on that area. The foam is subsequently rinsed away with clean water.

## EXAMPLES

The invention is further illustrated by reference to the following examples.

### Example 1

This Example illustrates a body cleansing liquid product suitable for use in the shower.

The product contains the following ingredients:

| Ingredients | % w/w |
|---|---|
| triethanolammonium n-butyl lauryl phosphate | 36 |
| preservative | 2 |
| water | to 100 |

This product can be used for cleansing the whole body surface, including the hair, for example under the shower, a convenient amount of say 5ml being placed in the palm of the hand prior to distributing over the body surface with added water to create a lather with superior volume and creaminess characteristics.

Examples 2 to 5 illustrate body shampoos for use in the shower or when bathing.

Example 2

| | % w/w |
|---|---|
| triethanolammonium n-hexyl-decyl phosphate | 36 |
| triethanolammonium laurate | 1 |
| triethanolammonium myristate | 2 |
| alkyldiaklylamine oxide (EMPIGEN OB: 30% active) | 8.3 |
| myristyldiethylamine oxide | 0.5 |
| cationised cellulose | 0.1 |
| propylene glycol | 10 |
| water | to 100 |

Example 3

| | % w/w |
|---|---|
| sodium ethyl-myristyl phosphate | 24 |
| lauryl dimethylbetaine | 3 |
| ethylene glycol monostearate | 1.5 |
| propylene glycol | 2.5 |
| preservative, perfume, dyes | q.v. |
| water | to 100% |

Example 4

| | % w/w |
|---|---|
| sodium methyl-lauryl phosphate | 30 |
| triethanolammonium laurate | 1 |
| triethanolammonium myristate | 2 |
| alkyldialkylamine oxide (EMIGEN OB 30% active) | 8.3 |
| cationised cellulose | 0.1 |
| propylene glycol | 10 |
| water | to 100 |

Example 5

| | % w/w |
|---|---|
| sodium n-octenyl-n-nonenyl phosphate | 20 |
| lauryl dimethylbetaine | 3 |
| ethylene glycol monostearate | 1.5 |
| propylene glycol | 2.5 |
| preservative, perfume, dyes | q.v. |
| water | to 100% |

Example 6

| | % w/w |
|---|---|
| sodium ethyl-lauryl phosphate | 30 |
| triethanolammonium laurate | 1 |
| triethanolammonium myristate | 2 |
| alkyldialkylamine oxide (EMIGEN OB 30% active) | 8.3 |
| cationised cellulose | 0.1 |
| propylene glycol | 10 |
| water | to 100 |

Example 7

| | % w/w |
|---|---|
| sodium ethyl-dodecenyl phosphate | 20 |
| lauryl dimethylbetaine | 3 |
| ethylene glycol monostearate | 1.5 |
| propylene glycol | 2.5 |
| preservative, perfume, dyes | q.v. |
| water | to 100% |

## Claims

1. An aqueous composition suitable for topical application to the skin or hair, which comprises:
(a) from 1 to 99.9% by weight of phosphate surfactant having the structure (1):

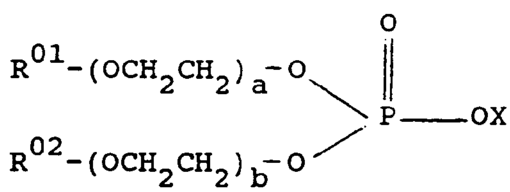

(1)

where $R^{01}$ and $R^{02}$ are different from each other and are each chosen from branched or-unbranched alkyl and alkenyl groups having an average of from 1 to 16 carbon atoms, the total number of carbon atoms present in the alkyl and alkenyl groups being from 10 to 20;
X is chosen from H, alkali metal, ammonium and substituted ammonium counterions; and
a and b are each chosen from 0 or a value of from 1 to 4;
but not including those phosphate surfactants which are n-hexyl-n-nonyl phosphate and n-hexyl-n-octyl phosphate; and
b. from 0.01 to 99% by weight of a cosmetically acceptable vehicle for the surfactant.

2. A composition according to claim 1, in which the dialkyl phosphate moiety of the alkyl phosphate salt is chosen from:
n-butyl-lauryl phosphate
n-hexyl-decyl phosphate
ethyl-myristyl phosphate
methyl-lauryl phosphate
n-octenyl-n-nonenyl phosphate
ethyl-lauryl phosphate, and
ethyl-dodecenyl phosphate.

3. A composition according to claim 1 or 2 in which the counterion is chosen from sodium, potassium or triethanolammonium.

4. A composition according to any preceding claim, in which the phosphate surfactant forms from 2 to 50% by weight of the composition.

5. A composition according to any preceding claim, which further comprises an anionic co-surfactant.

6. A composition according to claim 5, in which the anionic co-surfactant is a short chain phosphate surfactant having the structure (2a):

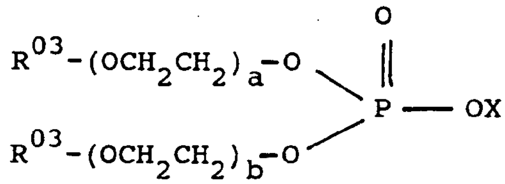

(2a)

where the $R^{03}$ groups are the same and are each chosen from branched or unbranched alkyl and alkenyl groups having an average of from 7 to 9 carbon atoms;

X is chosen from H, alkali metal ammonium and substituted ammonium counterions; and

a & b are each chosen from 0 or a value of from 1 to 4.

7. A composition according to claim 5 or 6, in which the phosphate moiety of the short chain phosphate surfactant is chosen from:
di-n-heptyl phosphate
di-n-octyl phosphate
di-n-nonyl phosphate
di-(diethyleneglycol-mono-n-nonyl ether) phosphate
di-(ethyleneglycol-mono-n-octyl ether) phosphate
di-n-heptenyl phosphate
di-n-octenyl phosphate
di-n-nonenyl phosphate
di-(triethyleneglycol-mono-n-octenyl ether) phosphate
di-(ethyleneglycol-mono-n-heptyl ether) phosphate
di-(7-methyloctyl) phosphate
di-(5-methylhexyl) phosphate
di-(6,6-dimethylheptyl) phosphate
n-hexyl-n-nonyl phosphate, and
n-hexyl-n-octyl phosphate.

8. A composition according to claims 6 or 7, in which the short chain phosphate surfactant forms from 2 to 50% by weight of the composition.

9. A composition according to claim 5, in which the anionic co-surfactant is a monoalkyl or monoalkenyl phosphate surfactant having the structure (2b):

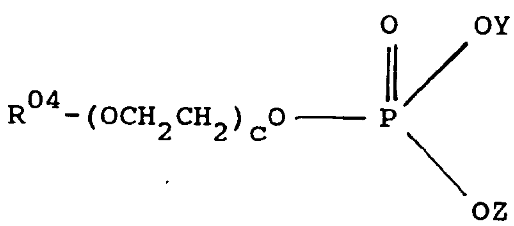

(2b)

where $R^{04}$ is chosen from branched or unbranched alkyl and alkenyl groups having from 6 to 18 carbon atoms;

Y and Z are each chosen from H, alkali metal, ammonium and substituted ammonium counterions; and

c is chosen from 0 or a value of from 1 to 4.

10. A composition according to claim 9, in which the phosphate moiety of the monoalkyl or monoalkenyl phosphate surfactant is chosen from:
mono-n-hexyl phosphate

mono-n-heptyl phosphate
mono-n-octyl phosphate
mono-n-nonyl phosphate
mono-n-decyl phosphate
mono-n-dodecyl phosphate (mono-lauryl phosphate)
mono-n-tetradecyl phosphate (monomyristyl phosphate)
mono-n-hexadecyl phosphate
mono-n-octadecyl phosphate
mono-(diethyleneglycol-mono-n-nonyl ether) phosphate
mono-(ethyleneglycol-mono-n-octyl ether) phosphate
mono-n-hexenyl phosphate
mono-n-heptenyl phosphate
mono-n-octenyl phosphate
mono-n-nonenyl phosphate
mono-n-dodecenyl phosphate
mono-(triethyleneglycol-mono-n-octenyl ether) phosphate
mono-(ethyleneglycol-mono-n-heptenyl ether) phosphate
mono-7-methyloctyl phosphate
mono-5-methylhexyl phosphate
mono-6,6-dimethylheptyl phosphate
mono-(ethyleneglycol-mono-n-octadecyl ether) phosphate
mono-(diethyleneglycol-mono-n-octadecenyl ether) phosphate
mono-(polyethyleneglycol[5EO]-monooleyl ether) phosphate, and
mono-(polyethyleneglycol[3EO]-monolauryl ether) phosphate.

**11.** A composition according to claims 9 or 10, in which the monoalkyl or monoalkenyl phosphate surfactant forms from 2 to 50% by weight of the composition.

**12.** A composition according to claim 5, in which the anionic co-surfactant is a long chain dialkyl or dialkenyl phosphate surfactant having the structure (2c):

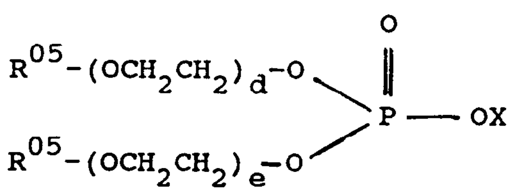

(2c)

where the $R^{05}$ groups are the same and are each chosen from branched or unbranched alkyl and alkenyl groups having an average of from 10 to 18 carbon atoms;
X is chosen from H, alkali metal ammonium and substituted ammonium counterions; and
d and e are each chosen from 0 or a value of from 1 to 10.

**13.** A composition according to claim 12, in which the phosphate moiety of the long chain dialkyl or dialkenyl phosphate surfactant is chosen from:
di-n-decyl phosphate
di-n-dodecyl phosphate (dilauryl phosphate)
di-n-tetradecyl phosphate (dimyristyl phosphate)
di-n-hexadecyl phosphate
di-n-octadecyl phosphate
di-n-dodecenyl phosphate
di-(ethyleneglycol-mono-n-octadecenyl ether) phosphate
di-(diethyleneglycol-mono-n-octadecenyl ether) phosphate
di-(polyethyleneglycol[5EO]-monooleyl ether) phosphate, and
di-(polyethyleneglycol[3EO]-monolauryl ether) phosphate.

**14.** A composition according to claims 12 or 13, in which the long chain dialkyl or dialkenyl phosphate surfactant forms from 1 to 10% by weight of the composition.

**15.** A composition according to any preceding claim, which further comprises a nonionic co-surfactant.

**16.** A composition according to any preceding claim, which further comprises a zwitterionic co-surfactant.

**17.** A composition according to any preceding claim, which is a liquid or gel product.

**18.** A composition according to claim 17, which is a washing product suitable for topical application to the face.

**19.** A composition according to any of claims 1 to 17, which is a shampoo.

**20.** A composition according to any of claims 1 to 16, which is a powder.

**21.** A composition according to any of claims 1 to 16, which is a bar or tablet suitable for washing the skin.

**22.** A composition according to claim 21, which is a soap bar or soap tablet.

**23.** A process for preparing a composition according to any preceding claim, which process comprises the steps of:
(i) preparing a mixture comprising:
a. from 1 to 99.9 % by weight of phosphate surfactant having the structure (1):

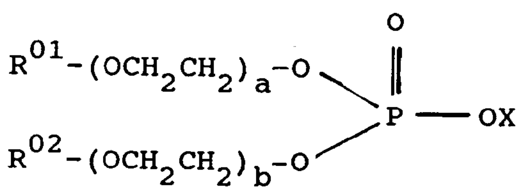

(1)

where $R^{01}$ and $R^{02}$ are different from each other and are each chosen from branched or unbranched alkyl and alkenyl groups having an average of from 1 to 16 carbon atoms, the total number of carbon atoms present in the alkyl and alkenyl groups being from 10 to 20;
X is chosen from H, alkali metal, ammonium and substituted ammonium counterions; and
a and b are each chosen from 0 or a value of from 1 to 4;
but not including those phosphate surfactants which are n-hexyl-n-nonyl phosphate and n-hexyl-n-octyl phosphate; and
b. from 0.01 to 99% by weight of a cosmetically acceptable vehicle for the surfactant, and
(ii) subsequently packaging the composition so formed into containers.

**24.** The use of a composition according to any of claims 1 to 22 for washing human shin or hair.

**Patentansprüche**

**1.** Wässerige Zusammensetzung, geeignet zur örtlichen Anwendung auf der Haut oder dem Haar, umfassend:
(a) 1 bis 99,9 Gew.-% eines Phosphattensids der Struktur (1):

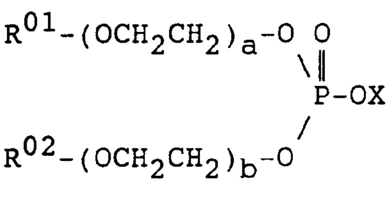

(1)

worin $R^{01}$ und $R^{02}$ voneinander verschieden sind und jeweils ausgewählt sind aus verzweigten

oder unverzweigten Alkyl- und Alkenylgruppen mit durchschnittlich 1 bis 16 Kohlenstoffatomen, wobei die Gesamtzahl an Kohlenstoffatomen, die in den Alkyl- und Alkenylgruppen vorliegt, von 10 bis 20 beträgt;

X ausgewählt ist aus H, Alkalimetall-, Ammonium- und substituierten Ammoniumgegenionen; und

a und b jeweils ausgewählt sind aus 0 oder einem Wert von 1 bis 4,

jedoch nicht jene Phosphattenside einschließend, die n-Hexyl-n-nonylphosphat und n-Hexyl-n-octyl-phosphat darstellen; und

(b) 0,01 bis 99 Gew.-% eines kosmetisch verträglichen Trägermittels für das Tensid.

2. Mittel nach Anspruch 1, wobei der Dialkylphosphatanteil des Alkylphosphatsalzes ausgewählt ist aus:
   n-Butyl-laurylphosphat
   n-Hexyl-decylphosphat
   Ethyl-myristylphosphat
   Methyl-laurylphosphat
   n-Octenyl-n-nonenylphosphat
   Ethyllaurylphosphat und
   Ethyldodecenylphosphat.

3. Mittel nach Anspruch 1 oder 2, wobei das Gegenion ausgewählt ist aus Natrium, Kalium oder Triethanolammonium.

4. Mittel nach einem vorangehenden Anspruch, wobei das Phosphattensid 2 bis 50 Gew.-% des Mittels ausmacht.

5. Mittel nach einem vorangehenden Anspruch, zusätzlich ein anionisches Co-Tensid umfassend.

6. Mittel nach Anspruch 5, wobei das anionische Co-Tensid ein kurzkettiges Phosphattensid der Struktur (2a) ist:

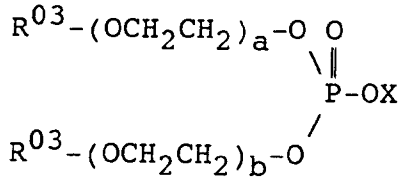

(2a)

   worin die $R^{03}$-Gruppen gleich sind und jeweils ausgewählt sind aus verzweigten oder unverzweigten Alkyl- und Alkenylgruppen mit durchschnittlich 7 bis 9 Kohlenstoffatomen;
   X ausgewählt ist aus H, Alkalimetall-, Ammonium- und substituierten Ammoniumgegenionen; und
   a und b jeweils ausgewählt sind aus 0 oder einem Wert von 1 bis 4.

7. Mittel nach Anspruch 5 oder 6, wobei der Phosphatanteil des kurzkettigen symmetrischen Phosphattensids ausgewählt ist aus:
   Di-n-heptylphosphat
   Di-n-octylphosphat
   Di-n-nonylphosphat
   Di-(diethylenglycol-mono-n-nonylether)phosphat
   Di-(ethylenglycol-mono-n-octylether)phosphat
   Di-n-heptenylphosphat
   Di-n-octenylphosphat
   Di-n-nonenylphosphat
   Di-(triethylenglycolmono-n-octenylether)phosphat
   Di-(ethylenglycol-mono-n-heptylether)phosphat
   Di-(7-methyloctyl)phosphat
   Di-(5-methylhexyl)phosphat
   Di-(6,6-dimethylheptyl)phosphat
   n-Hexyl-n-nonylphosphat und

n-Hexyl-n-octylphosphat.

8. Mittel nach Anspruch 6 oder 7, wobei das kurzkettige Phosphattensid 2 bis 50 Gew.-% des Mittels ausmacht.

9. Mittel nach Anspruch 5, wobei das anionische Co-Tensid ein Monoalkyl- oder ein Monoalkenylphosphattensid der Struktur (2b) ist:

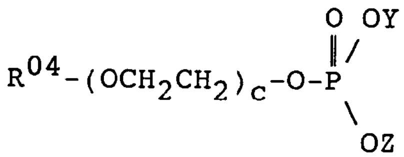

(2b)

worin $R^{04}$ ausgewählt ist aus verzweigten oder unverzweigten Alkyl- und Alkenylgruppen mit 6 bis 18 Kohlenstoffatomen;
Y und Z jeweils ausgewählt sind aus H, Alkalimetall-, Ammonium- und substituierten Ammoniumgegenionen; und
c ausgewählt ist aus 0 oder einem Wert von 1 bis 4.

10. Mittel nach Anspruch 9, wobei der Phosphatanteil des Monoalkyl- oder Monoalkenylphosphattensids ausgewählt ist aus:
Mono-n-hexylphosphat
Mono-n-heptylphosphat
Mono-n-octylphosphat
Mono-n-nonylphosphat
Mono-n-decylphosphat
Mono-n-dodecylphosphat(monolaurylphosphat)
Mono-n-tetradecylphosphat(monomyristylphosphat)
Mono-n-hexadecylphosphat
Mono-n-octadecylphosphat
Mono-(diethylenglycol-mono-n-nonylether)phosphat
Mono-(ethylenglycol-mono-n-octylether)phosphat
Mono-n-hexenylphosphat
Mono-n-heptenylphosphat
Mono-n-octenylphosphat
Mono-n-nonenylphosphat
Mono-n-dodecenylphosphat
Mono-(triethylenglycol-mono-n-octenylether)phosphat
Mono-(ethylenglycol-mono-n-heptenylether)phosphat
Mono-7-methyloctylphosphat
Mono-5-methylhexylphosphat
Mono-6,6-dimethylheptylphosphat
Mono-(ethylenglycol-mono-n-octadecylether)phosphat
Mono-(diethylenglycol-mono-n-octadecenylether)phosphat
Mono-(polyethylenglycol[5EO]monooleylether)phosphat und
Mono-(polyethylenglycol[3EO]monolaurylether)phosphat.

11. Mittel nach Anspruch 9 oder 10, wobei das Monoalkyl- oder Monoalkenylphosphattensid 2 bis 50 Gew.-% des Mittels ausmacht.

12. Mittel nach Anspruch 5, wobei das anionische Co-Tensid ein langkettiges Dialkyl- oder Dialkenylphosphattensid der Struktur (2c) ist:

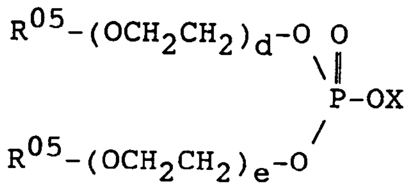

(2c)

worin die $R^{05}$-Gruppen gleich sind und jeweils ausgewählt sind aus verzweigten oder unverzweigten Alkyl- und Alkenylgruppen mit durchschnittlich 10 bis 18 Kohlenstoffatomen;

X ausgewählt ist aus H, Alkalimetall-, Ammonium- und substituierten Ammoniumgegenionen; und

d und e jeweils ausgewählt sind aus 0 oder einem Wert von 1 bis 10.

**13.** Mittel nach Anspruch 12, wobei der Phosphatanteil des langkettigen Dialkyl- oder Dialkenylphosphattensids ausgewählt ist aus:

Di-n-decylphosphat
Di-n-dodecylphosphat(dilaurylphosphat)
Di-n-tetradecylphosphat(dimyristylphosphat)
Di-n-hexadecylphosphat
Di-n-octadecylphosphat
Di-n-dodecenylphosphat
Di-(ethylenglycol-mononoctadecenylether)phosphat
Di-(diethylenglycol-mono-n-octadecenylether)phosphat
Di-(polyethylenglycol[5EO]monooleylether)phosphat
Di-(polyethylenglycol[3EO]monolaurylether)phosphat

**14.** Mittel nach den Ansprüchen 12 oder 13, wobei das langkettige Dialkyl- oder Dialkenylphosphattensid 1 bis 10 Gew.-% des Mittels ausmacht.

**15.** Mittel nach einem vorangehenden Anspruch, zusätzlich ein nichtionisches Co-Tensid umfassend.

**16.** Mittel nach einem vorangehenden Anspruch, zusätzlich ein zwitterionisches Co-Tensid umfassend.

**17.** Mittel nach einem vorangehenden Anspruch, nämlich ein flüssiges oder gelförmiges Produkt.

**18.** Mittel nach Anspruch 17, nämlich ein Waschprodukt, geeignet zur örtlichen Anwendung im Gesicht.

**19.** Mittel nach einem der Ansprüche 1 bis 17, nämlich ein Shampoo.

**20.** Mittel nach einem der Ansprüche 1 bis 16, nämlich ein Pulver.

**21.** Mittel nach einem der Ansprüche 1 bis 16, nämlich ein Riegel oder eine Tafel, geeignet zum Waschen der Haut.

**22.** Mittel nach Anspruch 21, nämlich ein Seifenriegel oder eine Seifentafel.

**23.** Verfahren zur Herstellung eines Mittels nach einem vorangehenden Anspruch umfassend die Schritte:

i. Herstellen eines Gemisches, umfassend

a. 1 bis 99,9 Gew.-% eines Phosphattensids der Formel mit der Struktur (1):

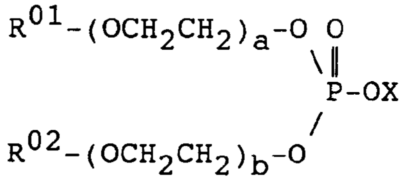

(1)

worin $R^{01}$ und $R^{02}$ voneinander verschieden sind und jeweils ausgewählt sind aus verzweigten oder unverzweigten Alkyl- und Alkenylgruppen mit durchschnittlich 1 bis 16 Kohlenstoffatomen,

wobei die Gesamtzahl an Kohlenstoffatomen, die in den Alkyl- und Alkenylgruppen vorliegt, von 10 bis 20 beträgt;

X ausgewählt ist aus H, Alkalimetall-, Ammonium- und substituierten Ammoniumgegenionen; und

a und b jeweils ausgewählt sind aus 0 oder einem Wert von 1 bis 4,

jedoch nicht jene Phosphattenside einschließend, die n-Hexyl-n-nonylphosphat und n-Hexyl-n-octyl-phosphat darstellen; und

(b) 0,01 bis 99 Gew.-% eines kosmetisch verträglichen Trägermittels für das Tensid, und

ii. anschließend Konfektionieren des so hergestellten Mittels in Behältern.

**24.** Verwendung eines Mittels nach einem der Ansprüche 1 bis 22 zum Waschen menschlicher Haut oder Haaren.

## Revendications

**1.** Composition aqueuse appropriée pour des applications locales à la peau ou aux cheveux, qui comprend:
(a) de 1 à 99,9% en poids d'un tensioactif phosphaté de formule (1) :

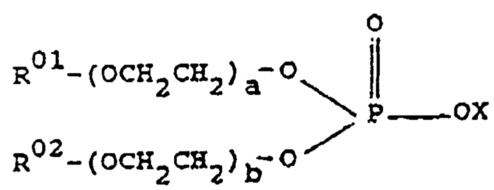

(1)

dans laquelle $R^{01}$ et $R^{02}$ qui sont différents l'un de l'autre, représentent chacun un radical alkyle ou alcényle ramifié ou non ramifié contenant chacun de 1 à 16 atomes de carbone en moyenne, le total des atomes de carbone dans les radicaux alkyle et alcényle étant compris entre 10 et 20 ;

X représente H ou un contre-ion de métal alcalin, d'ammonium ou d'ammonium substitué ; et

a et b représentent chacun 0 ou un nombre de 1 à 4 ;

mais sans incorporer les tensioactifs phosphatés qui sont des n-hexyl-n-nonyl-phosphates et des n-hexyl-n-octylphosphates ; et

(b) de 0,01 à 99% en poids d'un véhicule cosmétiquement acceptable pour le tensioactif.

**2.** Composition selon la revendication 1, dans laquelle le fragment dialkylphosphate est un alkylphosphate choisi parmi les suivants :

n-butyl-laurylphosphate
n-hexyl-décylphosphate
éthyl-myristylphosphate
méthyl-laurylphosphate
n-octényl-n-nonénylphosphate
éthyl-laurylphosphate, et
éthyl-dodécénylphosphate

**3.** Composition selon la revendication 1 ou 2, dans laquelle le contre-ion est l'ion sodium, potassium, ou triéthanolammonium.

**4.** Composition selon l'une quelconque des revendications précédentes, dans laquelle le tensioactif phosphaté forme de 2 à 50% en poids de la composition.

**5.** Composition selon l'une quelconque des revendications précédentes, qui comprend en outre un co-tensioactif anionique.

**6.** Composition selon la revendication 5, dans laquelle le co-tensioactif anionique est un tensioactif phosphaté à chaîne courte de formule (2a) :

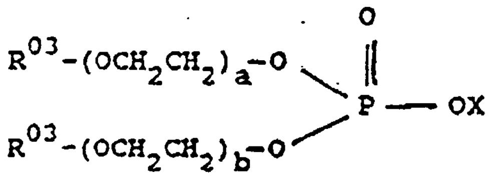

(2a)

dans laquelle les groupes $R^{03}$ sont identiques et chacun représente un radical alkyle ou alcényle ramifié ou non ramifié et contenant en moyenne de 7 à 9 atomes de carbone ;
X représente H ou un contre-ion métal alcalin, ammonium ou ammonium substitué ; et
a et b représente chacun 0 ou un nombre de 1 à 4.

7. Composition selon la revendication 5 ou 6, dans laquelle le fragment phosphate du tensioactif phosphaté à chaîne courte est choisi parmi les suivants :
   di-n-heptylphosphate
   di-n-octylphosphate
   di-n-nonylphosphate
   di(éther mono-n-nonylique de diéthylèneglycol) phosphate
   di(éther mono-n-octylique d'éthylèneglycol) phosphate
   di-n-hepténylphosphate
   di-n-octénylphosphate
   di-n-nonénylphosphate
   di-(éther mono-n-octénylique de triéthylèneglycol)phosphate
   di-(éther mono-n-heptylique d'éthylèneglycol) phosphate
   di-(7-méthyloctyl)phosphate
   di-(5-méthylhexyl)phosphate
   di-(6,6-diméthylheptyl)phosphate
   n-hexyl-n-nonylphosphate, et
   n-hexyl-n-octyl-phosphate.

8. Composition selon la revendication 6 ou 7, dans laquelle le tensioactif phosphaté à chaîne courte forme de 2 à 50% en poids de la composition.

9. Composition selon la revendication 5, dans lequel le co-tensioactif anionique est un tensioactif monoalkyl- ou monoalcénylphosphaté de structure (2b) :

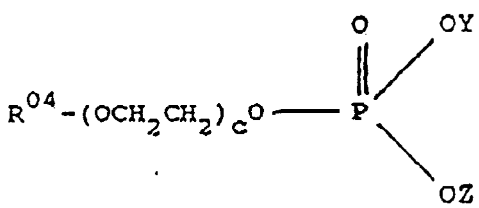

(2b)

dans laquelle $R^{04}$ est un radical alkyle ou alcényle ramifié ou non ramifié de 6 à 18 atomes de carbone ;
Y et Z représentent chacun H ou un contre-ion métal alcalin, ammonium ou ammonium substitué ; et
c représente 0 ou un nombre de 1 à 4.

10. Composition selon la revendication 9, dans laquelle le fragment phosphate du tensioactif monoalkyl- ou monoalcényl- phosphaté est choisi parmi les suivants :
   mono-n-hexylphosphate
   mono-n-heptylphosphate
   mono-n-octylphosphate
   mono-n-nonylphosphate
   mono-n-décylphosphate
   mono-n-dodecylphosphate (mono-laurylphosphate)

mono-n-tetradécylphosphate (monomyristylphosphate)
mono-n-hexadécylphosphate
mono-n-octadécylphosphate
mono-(éther mono-n-nonylique de diéthylèneglycol)phosphate
mono-(éther mono-n-octylique d'éthylèneglycol)phosphate
mono-n-hexényl-phosphate
mono-n-hepténylphosphate
mono-n-octényl-phosphate
mono-n-nonényl-phosphate
mono-n-dodecényl-phosphate
mono-(éther mono-n-octénylique de triéthylèneglycol)phosphate
mono-(éther mono-n-hepténylique d'éthylèneglycol)phosphate ; mono-5-méthylhexylphosphate
mono-7-méthyloctyl-phosphate
mono-6,6-diméthylheptylphosphate
mono-(éther n-octadécylique d'éthylèneglycol) phosphate
mono-(éther mono-n-octadécénylique de diéthylèneglycol)phosphate
mono-(éther mono-oléylique de polyéthylèneglycol 5OE)phosphate, et
mono-(éther monolaurylique de polyéthylèneglycol 3 OE)phosphate.

**11.** Composition selon la revendication 9 ou 10, dans laquelle le tensioactif monoalkyl- ou monoalcénylphosphaté forme de 2 à 50% en poids de la composition.

**12.** Composition selon la revendication 5, dans laquelle le co-tensioactif anionique est un tensioactif dialkyl- ou dialcényl- phosphaté à chaîne longue de structure (2c) :

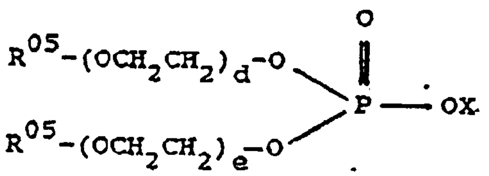

(2c)

dans laquelle les groupes $R^{05}$ sont identiques et chacun représente un radical alkyle ou alcényle ramifié ou non ramifié contenant en moyenne de 10 à 18 atomes de carbone ;
X représente H ou un contre-ion de métal alcalin, d'ammonium ou d'ammonium substitué ; et
d et e représentent chacun 0 ou un nombre de 1 à 10.

**13.** Composition selon la revendication 12, dans laquelle le fragment phosphate du tensioactif dialkyl- ou dialcényl- phosphaté à chaîne longue est choisi parmi les suivants :
di-n-décylphosphate
di-n-dodecylphosphate (dilauryl-phosphate)
di-n-tetradécylphosphate (dimyristyl-phosphate)
di-n-hexadécylphosphate
di-n-octadécylphosphate
di-n-dodecényl-phosphate
di-(éther mono-n-octadécénylique d'éthylèneglycol)phosphate
di-(éther mono-n-octadécénylique de diéthylèneglycol)phsophate
di-(éther mono-oléylique de polyéthylèneglycol 5 OE)phosphate
di-(éther monolaurylique de polyéthylèneglycol 3 OE)phosphate.

**14.** Composition selon la revendication 12 ou 13, dans laquelle le tensioactif dialkyl- ou dialcénylphosphaté à chaîne longue forme de 1 à 10% en poids de la composition.

**15.** Composition selon l'une quelconque des revendications précédentes qui comprend en outre un co-tensioactif non ionique.

**16.** Composition selon l'une quelconque des revendications précédentes, qui contient en outre un co-tensioactif zwitterionique.

**17.** Composition selon l'une quelconque des revendications précédentes, qui est un produit liquide ou en gel.

**18.** Composition selon la revendication 17, qui est un produit lavant qui convient à une application locale sur le visage.

**19.** Composition selon l'une quelconque des revendications 1 à 17, qui est un schampooing.

**20.** Composition selon l'une quelconque des revendications 1 à 16, qui est une poudre.

**21.** Composition selon l'une quelconque des revendications 1 à 16, qui est un pain ou une pastille permettant le lavage de la peau.

**22.** Composition selon la revendication 21, qui est un pain de savon ou une pastille de savon.

**23.** Procédé de préparation d'une composition selon l'une quelconque des revendications précédentes, procédé qui consiste :
i. à préparer un mélange comprenant :
(a) de 1 à 99,9% en poids d'un tensioactif phosphaté de formule (1) :

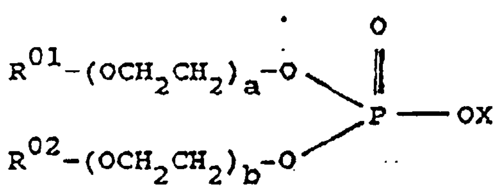

(1)

dans laquelle $R^{01}$ et $R^{02}$ qui sont différents l'un de l'autre, représentent chacun un radical alkyle ou alcényle ramifié ou non ramifié contenant chacun de 1 à 16 atomes de carbone en moyenne, le total des atomes de carbone dans les radicaux alkyle et alcényle étant compris entre 10 et 20 ;
X représente H ou un contre-ion de métal alcalin, d'ammonium ou d'ammonium substitué ; et
a et b représentent chacun 0 ou un nombre de 1 à 4 ;
mais sans incorporer les tensioactifs phosphatés qui sont des n-hexyl-n-nonyl-phosphates et des n-hexyl-n-octylphosphates ; et
(b) de 0,01 à 99% en poids d'un véhicule cosmétiquement acceptable pour le tensioactif,
ii. à conditionner ensuite la composition ainsi formée dans des récipients.

**24.** Utilisation d'une composition selon l'une quelconque des revendications 1 à 22, pour laver la peau et les cheveux humains.